# EUROPEAN PATENT SPECIFICATION

(11) **EP 4 378 385 B1**
(45) Date of publication and mention of the grant of the patent: **10.06.2026**
(21) Application number: 23211803.4
(22) Date of filing: 23.11.2023
(51) Int. Cl.: A61B 5/11, A61B 5/1455, A61B 5/00, A61B 5/024, A61B 5/026

(54) **AUTOMATIC MEASUREMENT SYSTEM FOR 6-MINUTE WALK TEST USING LIDAR SENSOR**
AUTOMATISCHES MESSSYSTEM FÜR 6-MINUTEN-GEHTEST MIT LIDAR-SENSOR
SYSTÈME DE MESURE AUTOMATIQUE POUR TEST DE MARCHE DE 6 MINUTES À L'AIDE D'UN CAPTEUR LIDAR

(30) Priority: 29.11.2022 KR 20220162281
(43) Date of publication of application: 05.06.2024
(73) Proprietor: Medical Solution System, Gimhae city, Gyeongsangnam-do 50834 (KR)
(72) Inventor: SEO, Joon Mo, 48270 Busan (KR); KANG, Minsoo, 47507 Busan (KR)
(74) Representative: M. Zardi & Co S.A.

(56) References cited:
- WO-A1-2021/132862
- CN-A- 113 229 809
- KR-A- 20210 058 283
- US-A1- 2011 152 726
- US-A1- 2022 331 028
- HOLLAND ANNE E. ET AL: "An official European Respiratory Society/American Thoracic Society technical standard: field walking tests in chronic respiratory disease", vol. 44, no. 6, 1 December 2014 (2014-12-01), GB, pages 1428 - 1446, XP093111429, ISSN: 0903-1936, Retrieved from the Internet <URL:https://erj.ersjournals.com/content/erj/44/6/1428.full.pdf> [retrieved on 202312], DOI: 10.1183/09031936.00150314
- SHINFENGD LIN ET AL: "Thermal face recognition under different conditions", BMC BIOINFORMATICS, BIOMED CENTRAL LTD, LONDON, UK, vol. 22, no. 5, 8 November 2021 (2021-11-08), pages 1 - 17, XP021298505, DOI: 10.1186/S12859-021-04228-Y
- ANONYMOUS SINEAD ET AL: "Six Minute Walk Test / 6 Minute Walk Test - Physiopedia", 8 March 2022 (2022-03-08), pages 1 - 8, XP093253165, Retrieved from the Internet <URL:https://web.archive.org/web/20220308005919/https://www.physio-pedia.com/Six_Minute_Walk_Test_/_6_Minute_Walk_Test>
- GONG WENJUAN ET AL: "Human action recognition based on estimated weak poses", EURASIP JOURNAL ON APPLIED SIGNAL PROCESSING, vol. 2012, no. 1, 25 July 2012 (2012-07-25), XP093303420, ISSN: 1687-6180, Retrieved from the Internet <URL:http://link.springer.com/content/pdf/10.1186/1687-6180-2012-162.pdf> DOI: 10.1186/1687-6180-2012-162
- QUOC-CUONG PHAM ET AL: "Real-Time Posture Analysis in a Crowd using Thermal Imaging", CVPR '07. IEEE CONFERENCE ON COMPUTER VISION AND PATTERN RECOGNITION; 18-23 JUNE 2007; MINNEAPOLIS, MN, USA, IEEE, PISCATAWAY, NJ, USA, 17 June 2007 (2007-06-17), pages 1 - 8, XP032392172, ISBN: 978-1-4244-1179-5, DOI: 10.1109/CVPR.2007.383496

## Description

### CROSS REFERENCE TO RELATED APPLICATION

The present application claims priority to Korean Patent Application No. 10-2022-0162281, filed November 29, 2022.

### BACKGROUND OF THE INVENTION

### Field of the Invention

The present disclosure relates to an automatic measurement system for a 6-minute walk test. More particularly, the present disclosure relates to an automatic measurement system for a 6-minute walk test using a LiDAR sensor.

### Description of the Related Art

The easiest way to assess patients' exercise capacity is to use a simple questionnaire about their exercise functions. However, this method relies on patients' subjective memory and is not an objective assessment method. Objective methods for evaluating exercise include stair climbing, a 6-minute walk test, a shuttle walk test, and an exercise stress test for cardiopulmonary function. Among these, the 6-minute walk test is widely used in many medical institutions as a simple and practical test that does not require special exercise equipment or specialized examiners, except for a 30-meter hallway.

The 6-minute walk test is a modified version of the 12-minute run test used for assessing exercise capacity for healthy people. The 6-minute walk test objectively evaluates a subject's exercise capacity by measuring the maximum distance (6 minute walk distance) the subject can walk in six minutes. Walking exercise is a relatively safe activity that most patients perform daily, and reflects the activity level of daily life well as walking involves submaximal exercise. In particular, the maximum distance that can be walked for six minutes is of significant importance as the maximum distance comprehensively reflects the overall and integrated response of all systems involved in walking exercise, such as cardiopulmonary function, systemic circulation, peripheral circulation, hemoglobin level, neuromuscular system, and muscle metabolism.

However, despite these advantages, the 6-minute walk test needs to be improved because the test has the following disadvantages. That is, in order to perform the 6-minute walk test as in the existing method, there are drawbacks such as requiring a relatively large space and a significant number of medical workers (medical workers to support or guide the patient, and medical workers to monitor the patient's movement). In addition, according to the existing 6-minute walk test method, it is difficult to accurately measure a patient's moving distance during measurement. Therefore, there is a need to develop a system that addresses the problems of the existing 6-minute walk test method, allowing a medical worker to measure a patient's walking more easily and measure and analyze the moving distance accurately.

As a related art of the present disclosure, Korean Patent Application Publication No. 10-2020-0052121 (titled: HEALTH MANAGEMENT SYSTEM USING SMART PHONE WITH 6-MINUTE WALK TEST APP, publication date: 14 May 2020) was proposed.

The foregoing is intended merely to aid in the understanding of the background of the present disclosure, and is not intended to mean that the present disclosure falls within the purview of the related art that is already known to those skilled in the art.

US2011152726A1 discloses a system for monitoring gait characteristics of a group of enrollees in a monitoring region of a public area, wherein the system includes an identifying device to enroll the group of enrollees into the system by measuring identifying information, and the system further includes a gait monitoring device to measure a sample gait characteristic during an enrollment phase, and to measure a gait characteristic of each enrollee subsequent to the enrollment phase. CN113229809A discloses a gait detection method comprising: acquiring environment information by using a laser radar; the environment information is the distance from the laser radar to the left leg of the tested person and the distance from the laser radar to the right leg of the tested person; performing data screening on the environment information, and determining screened data; capturing and tracking the leg information and the walking state of the tested person in real time according to the screened data; determining gait kinematics parameters according to the leg information and the walking state; the gait kinematics parameters comprise a step length, a stride, a step width, a gait period, swing time, standing time and double-support time. WO2021132862A1 describes a system related to the 6-minute walking test, that employs LiDAR sensors for predicting a subject's cardiopulmonary function from walking data. The system includes a gait meter with a display, memory, processor, motion sensor, and communication interface. The system also works with an external computing device, a server, and an oxygen saturation meter.

### SUMMARY OF THE INVENTION

The scope of protection of the present invention is limited by the appended claims. The present disclosure has been made keeping in mind the above problems occurring in the previously proposed methods, and the present disclosure is directed to providing an automatic measurement system for a 6-minute walk test using a LiDAR sensor, wherein when the 6-minute walk test is performed, the LiDAR sensor is placed in the space and a 6-minute walk test result is automatically measured, so that a medical worker can measure a patient's walking more easily and can measure and analyze the moving distance accurately.

In addition, the present disclosure is directed to providing an automatic measurement system for a 6-minute walk test using a LiDAR sensor, wherein a pulse oximeter worn on a patient performing the walk test is used to measure oxygen saturation and pulse, so that while the 6-minute walk test is performed, a medical worker can effectively monitor the patient's breathing condition.

In addition, the present disclosure is directed to providing an automatic measurement system for a 6-minute walk test using a LiDAR sensor, wherein a moving path and a moving distance are accurately analyzed using the LiDAR sensor, so compared to the existing walking test method, applying the way a pedestrian walks back and forth a short distance enables the 6-minute walk test to be performed in even smaller areas, especially, even in a non-straight space, thus maximizing the use of a space for performing the 6-minute walk test.

According to the present invention, there is provided an automatic measurement system for a 6-minute walk test using a LiDAR sensor,
the automatic measurement system including:
a body;
a LiDAR sensor part provided at one side of the body; and
a controller configured to perform control such that the pedestrian's moving distance is analyzed using the pedestrian's walking data collected by the LiDAR sensor part and the analyzed moving distance of the pedestrian is output, characterized by further comprising:
   an output part provided at one side of the body, and configured to output the pedestrian's moving distance;
   a communication part configured to communicate with a medical worker terminal, and provide the pedestrian's moving distance to the medical worker terminal;
   an infrared camera configured to detect the pedestrian's body heat,
   wherein the controller is configured to recognize that the pedestrian is a human, using the pedestrian's body heat detected by the infrared camera, thereby automating and processing measurement of the pedestrian's moving distance,
   wherein the LiDAR sensor part is configured to collect a pedestrian's walking data so that the pedestrian's moving distance is analyzed.

The LiDAR sensor part is configured to collect the walking data for analyzing the pedestrian's moving distance as well as the pedestrian's moving path, and
the controller is configured to perform control such that the pedestrian's moving distance as well as the pedestrian's moving path are analyzed using the pedestrian's walking data collected by the LiDAR sensor part and the analyzed moving distance and moving path of the pedestrian are output.

Preferably,
the LiDAR sensor part includes:
an optical transmission module configured to generate and emit a laser;
an optical reception module configured to focus optical signals scattered by the pedestrian's body; and
an optical detection module configured to detect the optical signals focused by the optical reception module,
wherein the controller is configured to analyze the pedestrian's moving distance using the detected optical signals.

The automatic measurement system further includes an output part provided at one side of the body, and configured to output the pedestrian's moving distance.

The automatic measurement system further includes a communication part configured to communicate with a medical worker terminal, and provide the pedestrian's moving distance to the medical worker terminal.

The automatic measurement system further includes a pulse oximeter configured to be worn on the pedestrian and measure the pedestrian's physiological data including oxygen saturation and pulse, and
wherein the controller is configured to receive the pedestrian's physiological data from the pulse oximeter and further analyze the pedestrian's breathing condition.

The automatic measurement system further includes: a camera configured to recognize the pedestrian's face and record the walking data on video; and
a voice support part configured to encourage the pedestrian or provide voice guidance.

The automatic measurement system further includes an infrared camera configured to detect the pedestrian's body heat,
wherein the controller is configured to recognize that the pedestrian is a human, using the pedestrian's body heat detected by the infrared camera, thereby automating and processing measurement of the pedestrian's moving distance.

According to the automatic measurement system, proposed in the present disclosure, for a 6-minute walk test using the LiDAR sensor, when the 6-minute walk test is performed, the LiDAR sensor is placed in the space and a 6-minute walk test result is automatically measured, so that a medical worker can measure a patient's walking more easily and can measure and analyze the moving distance accurately.

In addition, according to the automatic measurement system, proposed in the present disclosure, for a 6-minute walk test using the LiDAR sensor, the pulse oximeter worn on a patient performing the walk test is used to measure oxygen saturation and pulse, so that while the 6-minute walk test is performed, a medical worker can effectively monitor the patient's breathing condition.

In addition, according to the automatic measurement system, proposed in the present disclosure, for a 6-minute walk test using the LiDAR sensor, a moving path and a moving distance are accurately analyzed using the LiDAR sensor, so compared to the existing walking test method, applying the way a pedestrian walks back and forth a short distance enables the 6-minute walk test to be performed in even smaller areas, especially, even in a non-straight space, thus maximizing the use of a space for performing the 6-minute walk test.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a diagram illustrating a configuration of an automatic measurement system for a 6-minute walk test using a LiDAR sensor, according to an embodiment of the present disclosure;
FIG. 2 is a diagram illustrating an example of an automatic measurement system for a 6-minute walk test using a LiDAR sensor, according to an embodiment of the present disclosure;
FIG. 3 is a diagram illustrating an example in which an automatic measurement system for a 6-minute walk test using a LiDAR sensor collects a pedestrian's walking data, according to an embodiment of the present disclosure;
FIG. 4 is a diagram illustrating a detailed configuration of a LiDAR sensor part in an automatic measurement system for a 6-minute walk test using a LiDAR sensor, according to an embodiment of the present disclosure;
FIG. 5 is a diagram illustrating an example of outputting a 6-minute walk test result through an output part in an automatic measurement system for a 6-minute walk test using a LiDAR sensor, according to an embodiment of the present disclosure;
FIG. 6 is a diagram illustrating an example of outputting a 6-minute walk test result in the form of various graphs of (cumulative) moving distance for six minutes, moving distance per minute, walking speed, oxygen saturation, and pulse rate in an automatic measurement system for a 6-minute walk test using a LiDAR sensor, according to an embodiment of the present disclosure;
FIG. 7 is a diagram illustrating an example of communication between a medical worker terminal and an automatic measurement system for a 6-minute walk test using a LiDAR sensor, according to an embodiment of the present disclosure; and
FIG. 8 is a diagram illustrating an example of a pedestrian walking and wearing a pulse oximeter in an automatic measurement system for a 6-minute walk test using a LiDAR sensor, according to an embodiment of the present disclosure.

### DETAILED DESCRIPTION OF THE INVENTION

Hereinafter, preferred embodiments of the present disclosure will be described in detail with reference to the accompanying drawings such that the present disclosure can be easily embodied by those skilled in the art to which the present disclosure belongs. However, in describing the preferred embodiments of the present disclosure in detail, if it is decided that a detailed description of the known function or configuration related to the present disclosure makes the subject matter of the present disclosure unclear, the detailed description will be omitted. In addition, throughout the drawings, the same reference numerals are used for parts having similar functions and operations.

Throughout the specification, when a part is referred to as being "connected" to another part, it includes not only being "directly connected", but also being "indirectly connected" by interposing the other part therebetween. In addition, when a part "includes" an element, this means that it further includes other elements, but does not exclude other elements, unless specifically stated otherwise.

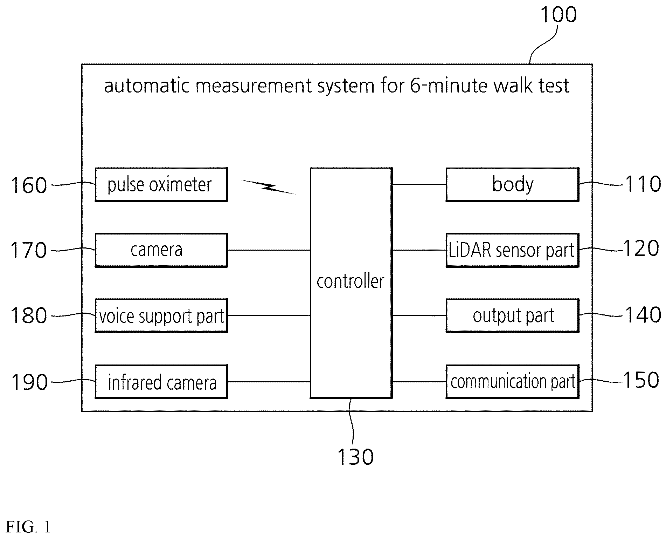

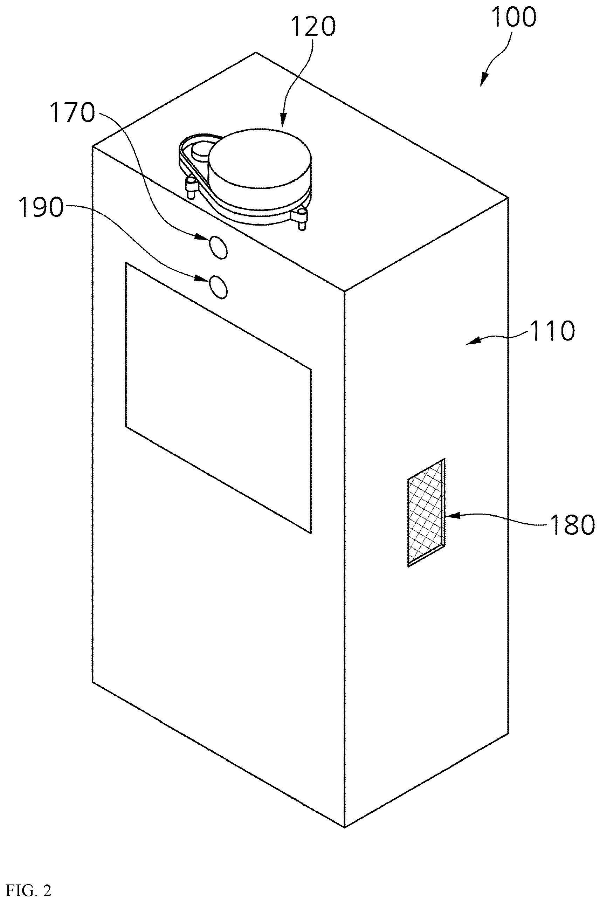

FIG. 1 is a diagram illustrating a configuration of an automatic measurement system for a 6-minute walk test using a LiDAR sensor, according to an embodiment of the present disclosure. FIG. 2 is a diagram illustrating an example of an automatic measurement system for a 6-minute walk test using a LiDAR sensor, according to an embodiment of the present disclosure. As shown in FIGS. 1 and 2, according to an embodiment of the present disclosure, an automatic measurement system 100 for a 6-minute walk test using a LiDAR sensor includes a body 110, a LiDAR sensor part 120, and a controller 130.

The automatic measurement system 100 further includes an output part 140, a communication part 150, a pulse oximeter 160, a camera 170, a voice support part 180, and an infrared camera 190.

The body 110 may be a frame constituting the shell of the automatic measurement system 100 for a 6-minute walk test using a LiDAR sensor, and a computer device including the controller 130 may be provided inside the body. The body 110 may have a general kiosk shape in the form of a a rectangular parallelepiped as shown in FIG. 2, but is not limited thereto, and may be realized in various forms or shapes. In particular, although not shown in the drawings, further including at least one auxiliary wheel for movement assistance provided at the bottom of the body 110, the automatic measurement system for the 6-minute walk test is realized to be mobile, so that the 6-minute walk test can be performed while the system moves to places without limitations as needed.

The LiDAR sensor part 120 is provided at one side of the body, and collects a pedestrian's walking data so that a moving distance of the pedestrian is analyzed. When the LiDAR sensor part 120 collects a pedestrian's walking data, the controller 130, which will be described later, analyzes the walking data and the pedestrian's moving distance for six minutes and outputs the distance. The LiDAR sensor part 120 also collects a pedestrian's walking data for analyzing the pedestrian's moving distance as well as his or her moving path. In this case, the controller 130 analyzes and outputs both the pedestrian's moving distance and the pedestrian's moving path.

According to an embodiment, a pedestrian's moving distance data described above includes not only the pedestrian's moving distance data, but also walking speed data and data of moving distance per unit time (for example, moving distance per minute).

A LiDAR sensor used in the LiDAR sensor part 120 of the automatic measurement system 100 for the 6-minute walk test using the LiDAR sensor, according to an embodiment of the present disclosure, will be described in more detail.

For the term "LiDAR sensor", LiDAR is an acronym of "light detection and ranging" or "laser imaging, detection, and ranging". This is a technology for obtaining 3D spatial information by measuring the time required for a laser beam to reflect off an object and return, using a high-output pulse laser. In particular, it is a technology that emits lasers to a target and detects the distance to an object and various properties, acting as the "eyes" of autonomous driving.

According to an embodiment of the present disclosure, an automatic measurement system 100 for a 6-minute walk test using a LiDAR sensor continuously measures the distance between a pedestrian and the automatic measurement system for the 6-minute walk test by using the LiDAR sensor, and accurately measures the pedestrian's moving track (moving path) and calculates the same backward to accurately measure the distance that the pedestrian travels for six minutes. Accordingly, when a pedestrian moves in a straight line as well as when the pedestrian moves in irregular directions and manners, such as zigzag walking, the pedestrian's moving path is accurately tracked, and thus the pedestrian's moving distance is accurately analyzed. By applying the same principle, applying the way a pedestrian walks back and forth a short distance enables the 6-minute walk test to be performed in even smaller areas, compared to the existing walking test method. In particular, this enables the 6-minute walk test to be performed even in a non-straight space, thus maximizing the use of a space for performing the 6-minute walk test. That is, applying the automatic measurement system, proposed in the present disclosure, for a 6-minute walk test using a LiDAR sensor enables the 6-minute walk test to be performed even in a narrow space including a curve.

In addition, in this process, a variety of data, such as a pedestrian's average moving speed, moving speed per minute, and moving distance per minute, may be extracted. Using the data enables additionally analyzing various types of information related to the pedestrian's exercise capacity and exercise state, in addition to simply calculating the pedestrian's moving distance.

In the meantime, there are various types of LiDAR sensors. As shown in FIG. 2, adopting a LiDAR sensor in a circular shape may be advantageous to track a moving path based on a fixed surrounding environment.

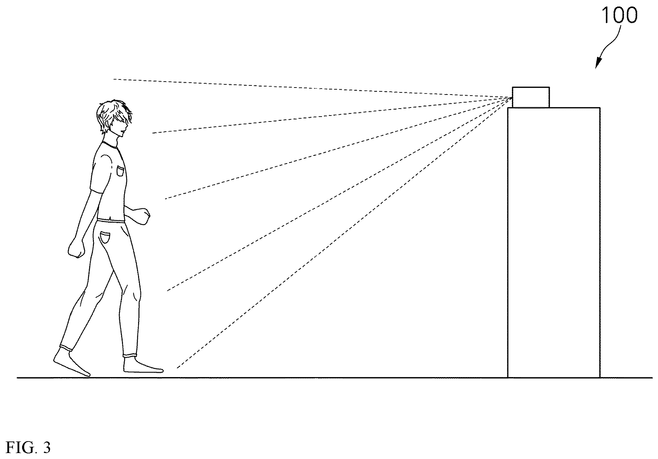

FIG. 3 is a diagram illustrating an example in which an automatic measurement system for a 6-minute walk test using a LiDAR sensor collects a pedestrian's walking data, according to an embodiment of the present disclosure. As shown in FIG. 3, according to the embodiment of the present disclosure, in the automatic measurement system 100 for the 6-minute walk test using the LiDAR sensor, the LiDAR sensor part 120 may be provided on the front upper portion of the body and may collect a walking pedestrian's walking data. However, the location at which the LiDAR sensor part 120 is provided is not limited to the front upper portion of the body, and may be provided at various locations and in various ways according to embodiments.

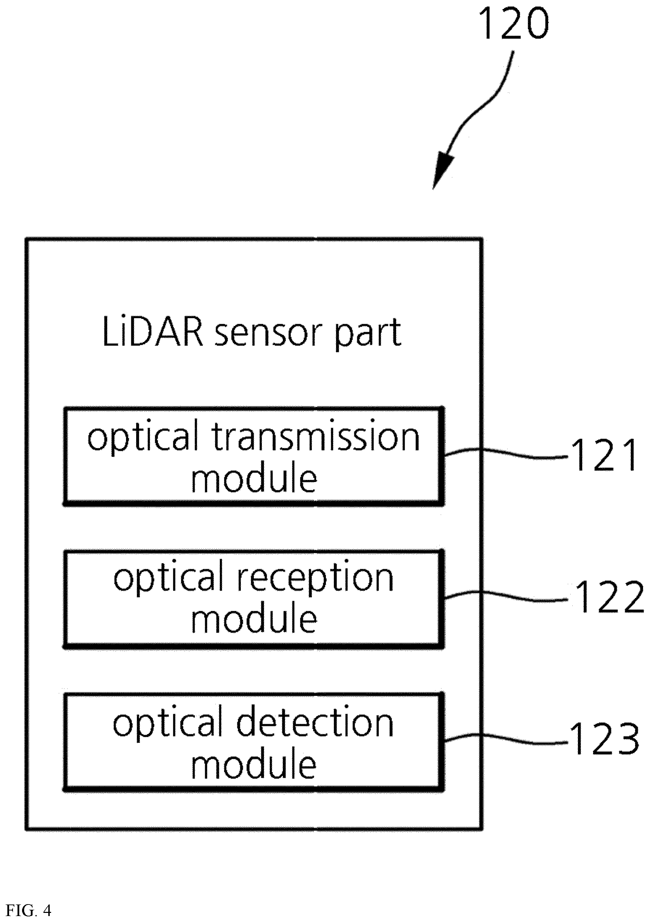

FIG. 4 is a diagram illustrating a detailed configuration of a LiDAR sensor part in an automatic measurement system for a 6-minute walk test using a LiDAR sensor, according to an embodiment of the present disclosure. As shown in FIG. 4, according to an embodiment of the present disclosure, the LiDAR sensor part 120 of the automatic measurement system 100 for the 6-minute walk test using the LiDAR sensor may include: an optical transmission module 121 for generating and emitting a laser; an optical reception module 122 for focusing optical signals scattered by a pedestrian's body; and an optical detection module 123 for detecting the optical signals focused by the optical reception module 122. The controller 130 may use the detected optical signals to analyze the pedestrian's moving distance.

The controller 130 performs control such that a pedestrian's moving distance is analyzed using the pedestrian's walking data collected by the LiDAR sensor part 120 and the analyzed moving distance of the pedestrian is output. In addition, according to an embodiment, by controlling or communicating with the output part 140, the communication part 150, the pulse oximeter 160, the camera 170, the voice support part 180, and the infrared camera 190, the controller 130 performs control such that various functions related to performing the 6-minute walk test by the automatic measurement system 100 for the 6-minute walk test using the LiDAR sensor are efflectively performed.

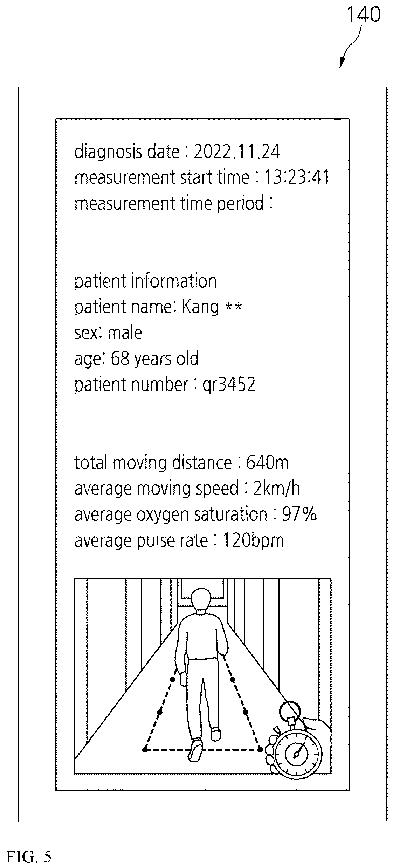

The output part 140 is provided at one side of the body 110, and outputs a pedestrian's moving distance. According to an embodiment of the present disclosure, the output part 140 of the automatic measurement system 100 for the 6-minute walk test using the LiDAR sensor may receive a selection signal of a user. FIG. 5 is a diagram illustrating an example of outputting a 6-minute walk test result through an output part in an automatic measurement system 100 for a 6-minute walk test using a LiDAR sensor, according to an embodiment of the present disclosure. Unless the size of the output part 140 is very small, the details output through the output part 140 do not need to be limited simply to a pedestrian's moving distance. That is, as shown in FIG. 5, according to an embodiment of the present disclosure, the output part 140 of the automatic measurement system 100 for the 6-minute walk test using the LiDAR sensor may output the following details: details of the test conduction time such as the diagnosis date of the walk test, the measurement start time, and the measurement time period; details of patient information such as a patient's name, sex, and age, and a patient number; and details of a test result such as the total moving distance, the average moving speed, the average oxygen saturation, and the average pulse rate for six minutes.

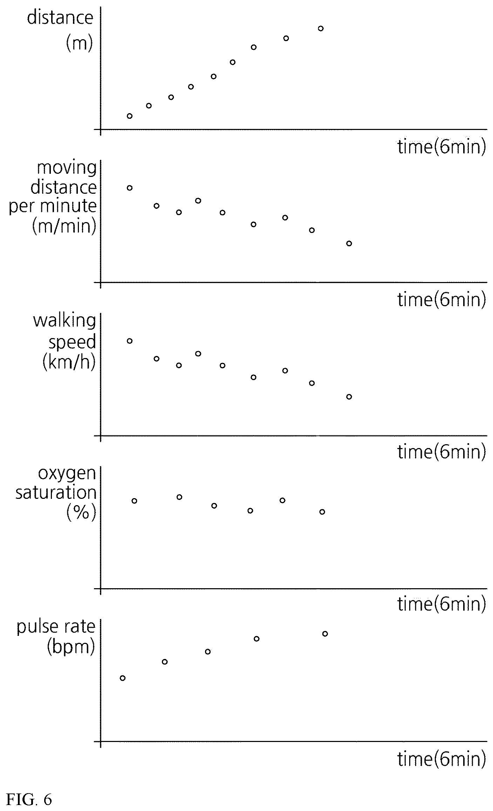

According to an embodiment, the output part 140 of the automatic measurement system 100 for the 6-minute walk test using the LiDAR sensor outputs the output details in the form of a graph, in addition to simply outputing the output details in text. This allows viewers to understand the output details much more quickly and directly. FIG. 6 is a diagram illustrating an example of outputting a 6-minute walk test result in the form of various graphs of (cumulative) moving distance for six minutes, moving distance per minute, walking speed, oxygen saturation, and pulse rate in an automatic measurement system 100 for a 6-minute walk test using a LiDAR sensor, according to an embodiment of the present disclosure.

In the meantime, as shown in FIG. 2, according to an embodiment of the present disclosure, in the automatic measurement system 100 for the 6-minute walk test using the LiDAR sensor, the output part 140 is generally provided on the front upper portion of the system and in the size of a display screen that a general kiosk provides, but the installation location or size of the output part 140 is not limited thereto. That is, according to an embodiment, the output part 140 may be positioned at the lateral surface or on the upper surface of the body instead of the front of the body depending on the size and the shape of the body. The size and the shape of the output part may be variously realized.

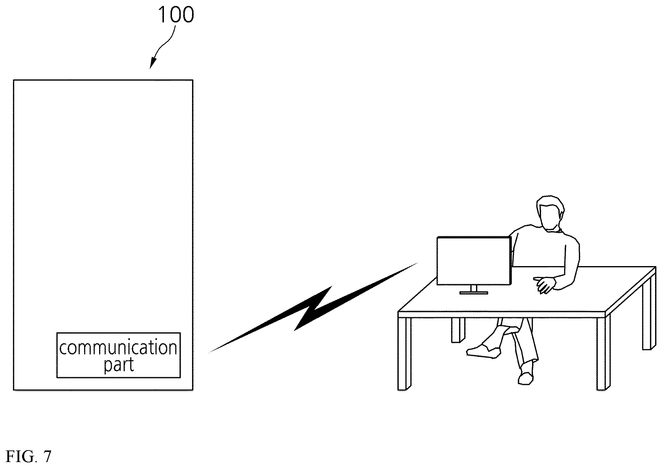

The communication part 150 communicates with a medical worker terminal, and provides the medical worker terminal with a pedestrian's moving distance. FIG. 7 is a diagram illustrating an example of communication between a medical worker terminal and an automatic measurement system for a 6-minute walk test using a LiDAR sensor, according to an embodiment of the present disclosure. As shown in FIG. 7, according to an embodiment of the present disclosure, the automatic measurement system 100 for the 6-minute walk test using the LiDAR sensor provides data, such as a pedestrian's moving distance, to a medical worker terminal located remotely, through the communication part 150. As a result, even if specialized medical workers, such as doctors, are not present near a pedestrian, a specialized 6-minute walk test service can be provided by a medical worker present remotely. Herein, it may be understood that the communication part 150 applies various wireless communication methods including Wireless Fidelity (Wi-Fi), a mobile radio communication network, a satellite network, Bluetooth, a wireless broadband internet (Wibro), 3/4/5th generation mobile telecommunication (3G/4G/5G), and long-term evolution (LTE) that enable Internet access.

Herein, the medical worker terminal may be realized as an electronic device that a medical worker uses. Examples of the electronic device may include at least one of the following: a smartphone, a tablet personal computer (PC), a mobile phone, a video phone, an e-book reader, a desktop PC, a laptop PC, a netbook computer, a workstation, a server, a personal digital assistant (PDA), a media box, a game console, an electronic dictionary, and a wearable device. Examples of the wearable device may include at least one of the following: an accessory type (e.g., a watch, ring, bracelet, anklet, necklace, glasses, contact lenses, or a head-mounted device (HMD)), a fabric or clothing-integrated type (e.g., electronic clothing), a body-attachable type (e.g., a skin pad or tattoo), and an implantable circuit. In various embodiments, the electronic device is not limited to the aforementioned devices, and may be a combination of two or more of the aforementioned various devices.

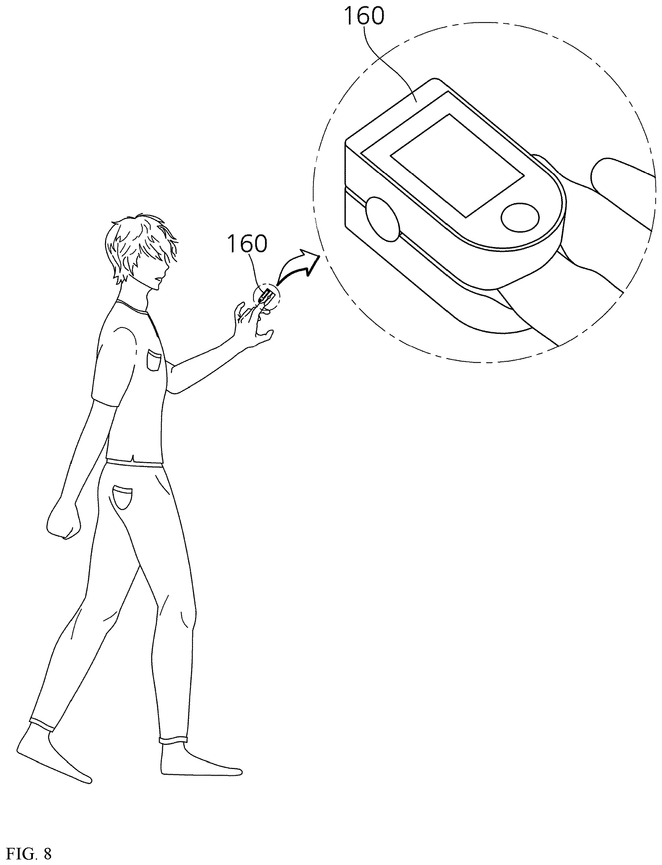

According to the present invention , the automatic measurement system 100 for the 6-minute walk test using the LiDAR sensor further includes a pulse oximeter 160 worn on a pedestrian to measure the pedestrian's physiological data including oxygen saturation and pulse. A pedestrian's physiological data received from the pulse oximeter 160 is transmitted to the controller 130, and is used to analyze the pedestrian's breathing condition. FIG. 8 is a diagram illustrating an example of a pedestrian walking and wearing a pulse oximeter in an automatic measurement system for a 6-minute walk test using a LiDAR sensor, according to an embodiment of the present disclosure. As shown in FIG. 8, according to an embodiment, a pedestrian walks while wearing a pulse oximeter on his or her finger, so a physiological signal during walking is measured and transmitted to the automatic measurement system 100, according to the present disclosure, for the 6-minute walk test using the LiDAR sensor. When the controller 130 determines that the analyzed breathing condition of the pedestrian is abnormal, this may be output through the output part 140 or may be reported as a warning sound through the voice support part 180, which will be described later, and may be reported to a medical worker terminal through the communication part 150.

According to the present invention, the automatic measurement system 100 for the 6-minute walk test using the LiDAR sensor further inlcudes: a camera 170 for recognizing a pedestrian's face and recording the walking data on video; and a voice support part 180 for encouraging a pedestrian or providing voice guidance. The camera 170 and the voice support part 180 may be installed in the location and in the method as shown in FIG. 2, but the installation location or installation method is not limited thereto.

In addition, according to the present invention, the automatic measurement system 100 for the 6-minute walk test using the LiDAR sensor further includes an infrared camera 190 for detecting a pedestrian's body heat. In this case, the controller 130 recognizes that the subject detected using the pedestrian's body heat detected by the infrared camera 190 is a human, thereby automating and processing the measurement of a pedestrian's moving distance for the 6-minute walk test. The infrared camera 190 may be installed in the location and in the method as shown in FIG. 2, but the installation location or installation method is not limited thereto.

As described above, according to an automatic measurement system, proposed in the present disclosure, for the 6-minute walk test using a LiDAR sensor, when the 6-minute walk test is performed, the LiDAR sensor is placed in the space and a 6-minute walk test result is automatically measured, so that a medical worker can measure a patient's walking more easily and can measure and analyze the moving distance accurately. The pulse oximeter worn on a patient performing the walk test is used to measure oxygen saturation and pulse, so that while the 6-minute walk test is performed, a medical worker can effectively monitor the patient's breathing condition. In addition, a moving path and a moving distance are accurately analyzed using the LiDAR sensor, so compared to the existing walking test method, applying the way a pedestrian walks back and forth a short distance enables the 6-minute walk test to be performed in even smaller areas, especially, even in a non-straight space, thus maximizing the use of a space for performing the 6-minute walk test.

The invention is limited by the scope of the appended claims.

## Claims

1. An automatic measurement system (100) for a 6-minute walk test using a LiDAR sensor, the automatic measurement system comprising:
a body (110);
a LiDAR sensor part (120) provided at one side of the body; a controller (130);
an output part (140) provided at one side of the body (110), and configured to output the pedestrian's moving distance;
a communication part (150) configured to communicate with a medical worker terminal, and provide the pedestrian's moving distance to the medical worker terminal;
a pulse oximeter (160) configured to be worn on the pedestrian and measure the pedestrian's physiological data including oxygen saturation and pulse; and
wherein the LiDAR sensor part (120) is configured to collect a pedestrian's walking data so that the pedestrian's moving distance as well as the pedestrian's moving path is analyzed,
wherein the controller (130) is configured to perform control such that the pedestrian's moving distance as well as the pedestrian's moving path are analyzed using the pedestrian's walking data collected by the LiDAR sensor part(120) and the analyzed moving distance and moving path of the pedestrian are output,
wherein the controller (130) is configured to receive the pedestrian's physiological data from the pulse oximeter (160) and further analyze the pedestrian's breathing condition, and
**characterized by** further comprising:
a voice support part (180) configured to encourage the pedestrian or provide voice guidance;
a camera (170) configured to recognize the pedestrian's face and record the walking data on video; and
an infrared camera (190) configured to detect the pedestrian's body heat, wherein the controller (130) is configured to recognize that the pedestrian is a human, using the pedestrian's body heat detected by the infrared camera (190), thereby automating and processing measurement of the pedestrian's moving distance.

## Patentansprüche

1. Automatisches Messsystem (100) für einen 6-Minuten-Gehtest unter Verwendung eines LiDAR-Sensors, wobei das automatische Messsystem umfasst:
einen Körper (110);
ein LiDAR-Sensorteil (120), das an einer Seite des Körpers vorgesehen ist;
eine Steuereinrichtung (130);
ein Ausgabeteil (140), das an einer Seite des Körpers (110) vorgesehen und dazu konfiguriert ist, die Bewegungsstrecke eines Fussgängers auszugeben;
ein Kommunikationsteil (150), das dazu konfiguriert ist, mit einem Terminal für medizinisches Personal zu kommunizieren, und die Bewegungsstrecke des Fussgängers dem Terminal für medizinisches Personal bereitzustellen;
ein Pulsoxymeter (160), das dazu konfiguriert ist, an dem Fussgänger getragen zu werden und die physiologischen Daten des Fussgängers umfassend Sauerstoffsättigung und Puls zu messen; und
wobei das LiDAR-Sensorteil (120) dazu konfiguriert ist, Gehdaten eines Fussgängers zu sammeln, so dass die Bewegungsstrecke des Fussgängers sowie der Bewegungsweg des Fussgängers analysiert wird,
wobei die Steuereinrichtung (130) dazu konfiguriert ist, eine Steuerung derart durchzuführen, dass die Bewegungsstrecke des Fussgängers sowie der Bewegungsweg des Fussgängers unter Verwendung der durch das LiDAR-Sensorteil (120) gesammelten Gehdaten des Fussgängers analysiert werden und die analysierte Bewegungsstrecke und der Bewegungspfad des Fussgängers ausgegeben werden,
wobei die Steuereinrichtung (130) dazu konfiguriert ist, die physiologischen Daten des Fussgängers von dem Pulsoxymeter (160) zu empfangen und ferner den Atmungszustand des Fussgängers zu analysieren, und
**dadurch gekennzeichnet, dass** es ferner umfasst:
ein Sprachunterstützungsteil (180), das dazu konfiguriert ist, den Fussgänger zu ermutigen oder eine Sprachanleitung bereitzustellen;
eine Kamera (170), die dazu konfiguriert ist, das Gesicht des Fussgängers zu erkennen und die Gehdaten auf Video aufzunehmen; und
eine Infrarotkamera (190), die dazu konfiguriert ist, die Körperwärme des Fussgängers zu detektieren, wobei die Steuereinrichtung (130) dazu konfiguriert ist, unter Verwendung der durch die Infrarotkamera (190) detektierten Körperwärme des Fussgängers zu erkennen, dass der Fussgänger ein Mensch ist, wodurch die Messung der Bewegungsstrecke des Fussgängers automatisiert und verarbeitet wird.

## Revendications

1. Système de mesure automatique (100) pour un test de marche de 6 minutes utilisant un capteur LiDAR, le système de mesure automatique comprenant :
un corps (110) ;
une partie de capteur LiDAR (120) disposée sur un côté du corps ;
une unité de commande (130) ;
une partie de sortie (140) disposée sur un côté du corps (110), et configurée pour délivrer en sortie la distance de déplacement du piéton ;
une partie de communication (150) configurée pour communiquer avec un terminal destiné au personnel médical, et fournir au terminal destiné au personnel médical la distance de déplacement du piéton ;
un oxymètre de pouls (160) configuré pour être porté par le piéton et mesurer les données physiologiques du piéton comprenant la saturation en oxygène et le pouls ; et
dans lequel la partie de capteur LiDAR (120) est configurée pour collecter des données de marche du piéton de sorte que la distance de déplacement du piéton ainsi que la trajectoire de déplacement du piéton soient analysées,
dans lequel l'unité de commande (130) est configurée pour exécuter une commande de sorte que la distance de déplacement du piéton ainsi que la trajectoire de déplacement du piéton soient analysées à l'aide des données de marche du piéton collectées par la partie de capteur LiDAR (120) et que la distance de déplacement et la trajectoire de déplacement analysées du piéton soient délivrées en sortie,
dans lequel l'unité de commande (130) est configurée pour recevoir les données physiologiques du piéton depuis l'oxymètre de pouls (160) et analyser en outre l'état respiratoire du piéton, et
**caractérisé en ce qu'**il comprend en outre :
une partie d'assistance vocale (180) configurée pour encourager le piéton ou fournir un guidage vocal ;
une caméra (170) configurée pour reconnaître le visage du piéton et enregistrer les données de marche sous forme vidéo ; et
une caméra infrarouge (190) configurée pour détecter la chaleur corporelle du piéton, dans lequel l'unité de commande (130) est configurée pour reconnaître que le piéton est un être humain, au moyen de la chaleur corporelle du piéton détectée par la caméra infrarouge (190), automatisant ainsi et traitant la mesure de la distance de déplacement du piéton.
